# EUROPEAN PATENT SPECIFICATION

(11) **EP 1 762 848 B1**
(45) Date of publication and mention of the grant of the patent: **12.11.2008**
(21) Application number: 05019716.9
(22) Date of filing: 09.09.2005
(51) Int. Cl.: G01N 33/487

(54) **Retracting mechanism for a bio reader**
Mechanismus zum Zurückziehen für einen Biosensor
Mécanisme de retrait pour un biocapteur.

(43) Date of publication of application: 14.03.2007
(73) Proprietor: Eumed Biotechnology Co., Ltd., Sanchung City Taipei Hsien (TW)
(72) Inventor: Chen, Tzer-Ming, Taipei Taiwan (CN); Yang, Lu-Hung, 802, Sec.1, Jhongyang Rd, Wuci Township Taichung Country Taiwan (CN)
(74) Representative: Casalonga, Axel

(56) References cited:
- EP-A- 1 321 769
- EP-A- 1 352 611
- EP-A- 1 480 037
- WO-A-20/04063747

## Description

The present invention relates to a retracting mechanism, and more particularly to a retracting mechanism for a bio reader able to read information of a strip inserted into the bio reader.

When taking a test with regard to physical conditions of a patient, a medical specialist manually transports a strip into a bio reader so that the bio reader is able to analyze the bio features contained on the strip so as to determine the physical conditions of the patient. While transporting the strip, inevitably, the medical specialist may contaminate the bio sample on the strip or the medical specialist may be infected by the bio sample. That is, medical specialists are constantly exposed to the threat of infection and the bio sample is also under the threat of being contaminated.

An other kind of bio reader is known from EP 1480037 and defines a measurement instrument for measuring glucose level in human blood having a complex structure comprising a hollow casing receiving therein a measurement circuit and a strip connector. The strip connector is adapted to receive therein a test strip and has a base mounted in the casing, a pusher movably connected to the base of the strip connector and having a ridge adapted for abutment of a strip, and a retracting finger securely connected to the pusher and slidably mounted on a side of the casing. The measurement circuit includes a CPU, a memory and is connected electrically to the strip connector.

To overcome the shortcomings, the present invention tends to provide an improved retracting mechanism for a bio reader to mitigate the aforementioned problems.

The primary objective of the present invention is to provide an improved retracting mechanism comprising a slidable button mounted on a side of the reader and having a linkage securely connected to a slider such that when the slidable button is moved, the slider is moved and the strip received inside the bio reader is retracted.

In one aspect of the present invention, the retracting mechanism further has a recoil spring to allow the slidable button to return to its original position after the slidable button is moved.

Other objects, advantages and novel features of the invention will become more apparent from the following detailed description when taken in conjunction with the accompanying drawings.
Fig. 1 is an exploded perspective view of the reader of the present invention;
Fig. 2 is a perspective view showing the assembled reader with the bottom cover removed;
Fig. 3 is a perspective view showing the assembled reader with the top cover and the bottom cover removed;
Fig. 4A is a schematic top plan view showing partial structural relationship inside the reader;
Fig. 4B is a schematic top plan view showing partial structural relationship inside the reader after the slidable button is moved by the recoil spring to its original position;
Fig. 5 is a schematic cross sectional view taken from line 5-5 in Fig. 4A; and
Fig. 6 is a schematic cross sectional view showing that the strip is easily removed from the reader.

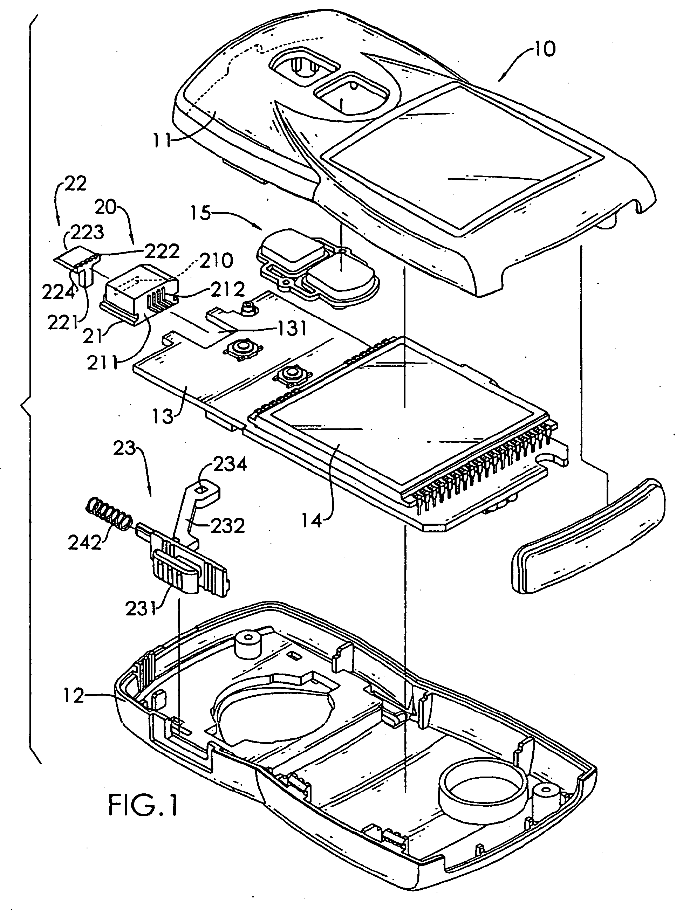

With reference to Fig. 1, it is noted that a bio reader in accordance with the present invention includes a hollow casing (10) composed of a top cover (11) and a bottom cover correlated with the top cover (11) to define therebetween a receiving space, a strip connector (20) firmly engaged with a peripheral edge of both the top cover (11) and the bottom cover (12) and a retracting finger (23) movably received in the casing (10) to drive a push (22) movably received in the strip connector (20).

Further, the casing (10) has a circuit board (13) electrically sandwiched between the strip connector (20), a monitor (14) connected to the circuit board (13) to display readings from the strip (not shown) and control buttons (15) mounted on top of the circuit board (13) and exposed from the top cover (11) for control of functions of the bio reader of the present invention. In order to mate the strip connector (20), the circuit board (13) has a cutout (131) defined in a peripheral edge thereof so that the strip connector (20) is able to be received in the cutout (131).

The strip connector (20) further has a base (21) mounted in the casing (10), an inlet (210) defined in a side face of the base (21) to receive therein a strip (not shown in this drawing), a sliding space (211) defined in a bottom face of the base (21), multiple contact legs (212) firmly received inside base (21) and extending out to connect the circuit board (13) and the push (22) movably connected to the base (21) and having an extension (221) extending downward to extending out of the sliding space (211) of the base (21), a ridge (222) formed on a top face of the push (22) and recesses (224) defined in the ridge (222).

The retracting finger (23) is slidably sandwiched between the top cover (11) and the bottom cover (12) and includes a slidable button (231), a linkage (232) extending from a side of the slidable button (231), a hole (234) defined in a free end of the linkage (232) and a guiding rod extending from the retracting finger (23) and is mounted to a compression spring (24).

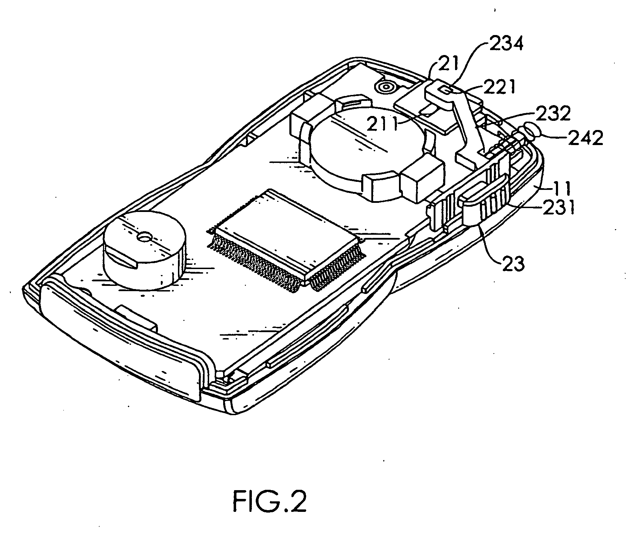

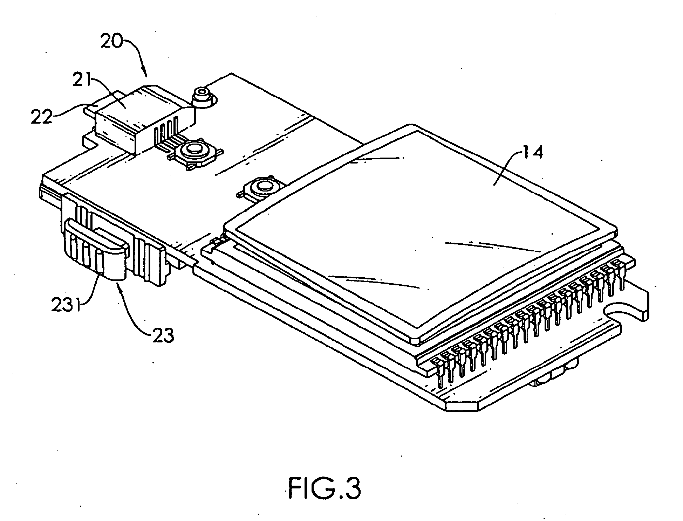

With reference to Figs. 2 and 3 and still using Fig. 1 for reference, when the bio reader of the present invention is assembled, the circuit board (13) and the monitor (14) are firmly received between the top cover (11) and the bottom cover (12). The strip connector (20) is securely received in the cutout (131) to have the contact legs (212) electrically connected to the circuit board (13) and the control buttons (15) are electrically mounted on the circuit board (13) and extended out of the top cover (11) so that an operator is able to use the control buttons (15) to command the circuit board (13) to proceed functions as required.

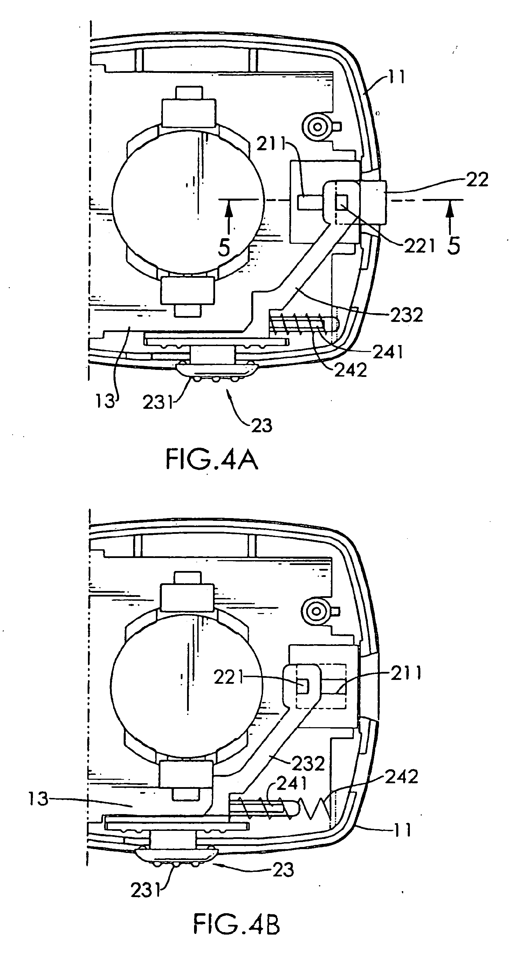

With reference to Figs. 4A, 4B, 5 and 6, it is noted that the push (22) is received in the strip connector (20) with the extension (221) extending out of the sliding space (211) such that a portion of the contact legs (212) received inside the strip connector (22) is in contact with a top face of the push (22). Then a free end of the extension (221) is extended into the hole (234) of the linkage (232) of the retracting finger (23). Finally the compression spring (242) is mounted around the guiding rod and abutted by a body of the retracting finger (23) and an inner side of the casing (10).

Therefore, it is seen that when the slidable button (231) is moved, due to the linkage (232) being securely connected to the extension (221) of the push (22), the push (22) is also moved.

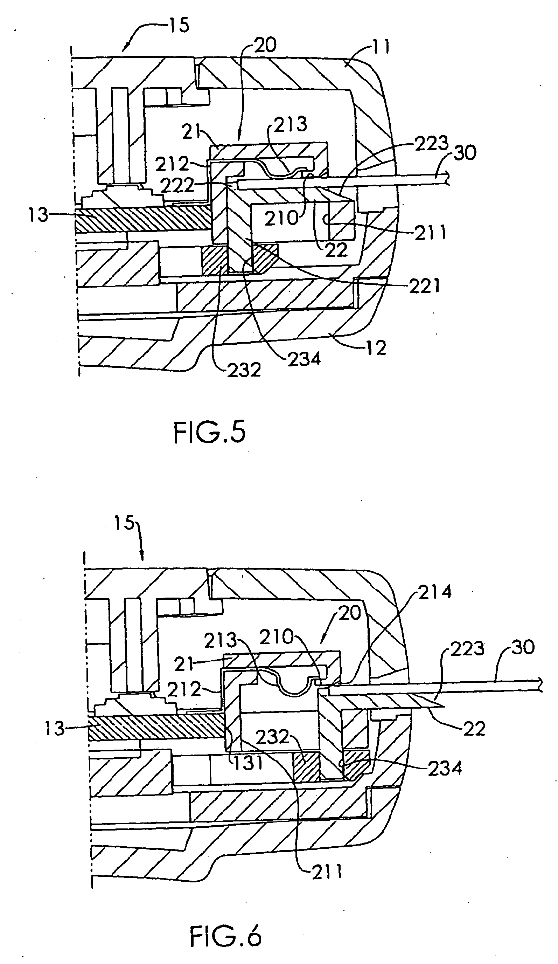

As shown in Figs. 5 and 6, after a strip (30) is inserted into the bio reader of the present invention from the inlet (210) and abutted by the ridge (222) of the push (22), arcuate portions (213) of the contact legs (212) are in contact with the strip (30) so that bio information contained on the strip (30) is read by the bio reader of the present invention. After the bio information is read, the operator is able to move the slidable button (231) toward the inlet (210) to allow the push (22) to be simultaneously moved toward the inlet (210). In the meantime, the recesses (224) in the ridge (222) move respectively through the arcuate portions (213) of the contact legs (212) so that the arcuate portions (213) of the contact legs (212) will not hinder movement of the push (22). It is noted that when the push (22) is moved, the strip (30) on top of the push (22) is also moved. When the slidable button (231) is moved toward the inlet (210), the compression spring (242) is compressed such that after the operator releases the slidable button (231), the recoil force from the compression spring (242) pushes the retracting finger (23) as well as the push (22) back to their original positions to be ready for the next process.

In order to facilitate the extension of the strip (30) into the bio reader of the present invention, an oblique face (214) is formed on a peripheral face defining the inlet (210) and the push (22) also has an inclined face (223).

As a conclusion, the retracting process of the strip (30) is entirely hand-free so that either the medical specialist or the bio sample on top of the strip is not affected and potential dangers of infection or contamination are avoided.

## Claims

1. A bio reader comprising a hollow easing (10) receiving therein a circuit board (13), a monitor (14) and a strip connector (20); **characterized in that**
the strip connector (20) is adapted to receive a strip therein and has
a base (21) mounted in the casing (10) comprising an inlet (210) to receive the strip therein
a push (22) movably received in the base (21) of the strip connector (20) and having a ridge (222) formed thereon and adapted for abutment of a strip on top of the push (22) and an extension (221) extending into the base (21); and
a retracting finger (23) securely connected to the extension (221) of the push (22) and slidably mounted on a side of the casing (10) such that movement of the retracting finger (23) is able to drive the push (22) to move in the base (21) toward the inlet (210) such that the strip on top of the push (22) is also moved; in order to retract said strip; and
the circuit board (13) is connected electrically to the monitor (14) and the strip connector (20).

2. The bio reader as claimed in claim 1, wherein the retracting finger (23) has a linkage (232) extending from the retracting finger (23) and having a hole (234) defined in a free end of the linkage (232) to correspond to and receive therein a free end of the extension (221) of the push (22) such that the movement of the retracting finger (23) is able to drive the push (22) to move.

3. The bio reader as claimed in claim 1, wherein the strip connector (20) has a sliding space (211) defined in a bottom face of the base (21) and the extension (221) of the push (22) is able to extend out of the sliding space (211).

4. The bio reader as claimed in claim 2, wherein the strip connector (20) has a sliding space (211) defined in a bottom face of the base (21) and the extension (211) of the push (22) is able to extend out of the sliding space (211) to connect to the linkage (232) so that the movement of the retracting finger (23) drives the push (22) to move.

5. The bio reader as claimed in claims 1 to 4, wherein the strip connector (20) has contact legs (212) composed of a portion extending out of the base (21) for electrical connection with the circuit board (13) and a portion received inside the base (21) for engagement with the strip and having an arcuate portion (213).

6. The bio reader as claimed in claim 5, wherein the push (22) has recesses (224) defined in the ridge (222) to facilitate the movement of the push (22) in relation to the contact legs (212).

7. The bio reader as claimed in claim 6 further comprising a compression spring (242) adapted to be abutted between an inner side of the casing (10) and the retracting finger (23) so that recoil force from the compression spring (242) due to the movement of the retracting finger (23) is able to return the retracting finger (23) to its original position.

8. The bio reader as claimed in claim 7, wherein the retracting finger (23) further has a guiding rod extending therefrom and the compression spring (242) is mounted around the guiding rod.

9. The bio reader as claimed in claim 8, wherein an oblique face (214) is formed on a peripheral face defining an inlet (210) defined in the base (21) of the strip connector (20) and an inclined face (223) is formed on the push (22) so that the extension of the strip into the bio reader is facilitated.

## Patentansprüche

1. Biosensor mit einem hohlen Gehäuse (10), in dem eine Leiterplatte (13), ein Monitor (14) und ein Streifenverbinder (20) aufgenommen sind; **dadurch gekennzeichnet, dass** der Streifenverbinder (20) einen Streifen darin aufnehmen kann und eine in dem Gehäuse (10) befestigte Basis (21) besitzt, die Folgendes umfasst:
einen Einlass (210), um den Streifen darin aufzunehmen;
einen Drücker (22), der beweglich in der Basis (21) des Streifenverbinders (20) aufgenommen ist und eine darauf ausgebildete Leiste (222) aufweist, die zum Anschlagen eines Streifens oben auf dem Drücker (22) dient, sowie einen Fortsatz (221), der sich in die Basis (21) hinein erstreckt; und
einen Rückholfinger (23), der fest mit dem Fortsatz (221) des Drückers (22) verbunden ist und verschieblich auf einer Seite des Gehäuses (10) gelagert ist, so dass durch die Bewegung des Rückholfingers (23) der Drücker (22) in der Basis (21) in Richtung zu dem Einlass (210) in Bewegung gesetzt werden kann, so dass der Streifen oben auf dem Drücker (22) ebenfalls in Bewegung gesetzt wird, um den Streifen zurückzuziehen; und
die Leiterplatte (13) elektrisch mit dem Monitor (14) und dem Streifenverbinder (20) verbunden ist.

2. Biosensor nach Anspruch 1, wobei der Rückholfinger (23) ein Verbindungselement (232) aufweist, das sich von dem Rückholfinger (23) erstreckt und eine in einem freien Ende des Verbindungselements (232) gebildete Bohrung (234) aufweist, die einem freien Ende des Fortsatzes (221) des Drückers (22) entspricht und dieses darin aufnimmt, so dass durch die Bewegung des Rückholfingers (23) der Drücker (22) in Bewegung gesetzt werden kann.

3. Biosensor nach Anspruch 1, wobei der Streifenverbinder (20) einen in einer Unterseite der Basis (21) gebildeten Schieberaum (211) aufweist und der Fortsatz (221) des Drückers (22) sich aus dem Schieberaum (211) heraus bewegen kann.

4. Biosensor nach Anspruch 2, wobei der Streifenverbinder (20) einen in einer Unterseite der Basis (21) gebildeten Schieberaum (211) aufweist und der Fortsatz (221) des Drückers (22) sich aus dem Schieberaum (211) heraus bewegen kann, um so mit dem Verbindungselement (232) verbunden zu werden, dass durch die Bewegung des Rückholfingers (23) der Drücker (22) in Bewegung gesetzt wird.

5. Biosensor nach Anspruch 1 bis 4, wobei der Streifenverbinder (20) Kontaktfüßchen (212) aufweist, die sich aus einem aus der Basis (21) zur elektrischen Verbindung mit der Leiterplatte (13) herausragenden Abschnitt und einem in der Basis (21) zum Eingriff in den Streifen aufgenommenen und einen gebogenen Abschnitt (213) aufweisenden Abschnitt zusammensetzen.

6. Biosensor nach Anspruch 5, wobei der Drücker (22) in der Leiste (222) gebildete Ausnehmungen (224) aufweist, um die Bewegung des Drückers (22) in Bezug auf die Kontaktfüßchen (212) zu erleichtern.

7. Biosensor nach Anspruch 6, der ferner eine Druckfeder (242) umfasst, die zwischen einer Innenseite des Gehäuses (10) und dem Rückholfinger (23) in Anschlag gebracht werden kann, so dass die infolge der Bewegung des Rückholfingers (23) von der Druckfeder (242) ausgehende Rückschnellkraft in der Lage ist, den Rückholfinger (23) in seine ursprüngliche Position zurückzubringen.

8. Biosensor nach Anspruch 7, wobei der Rückholfinger (23) ferner eine sich davon erstreckende Führungsstange aufweist und die Druckfeder (242) um die Führungsstange herum angebracht ist.

9. Biosensor nach Anspruch 8, wobei eine schräge Fläche (214) auf einer Umfangsfläche ausgebildet ist, die einen in der Basis (21) des Streifenverbinders (20) gebildeten Einlass (210) definiert, und eine geneigte Fläche (223) auf dem Drücker (22) ausgebildet ist, so dass sich der Streifen leichter in den Biosensor hinein bewegen kann.

## Revendications

1. Lecteur biologique comprenant un boîtier creux (10) dans lequel sont logés une carte de circuit (13), un moniteur (14) et un connecteur de bande (20) ;
**caractérisé en ce que** le connecteur de bande (20) est adapté pour recevoir une bande à l'intérieur et comporte :
une base (21) montée dans le boîtier (10) comprenant une entrée (210) destinée à recevoir la bande ;
un poussoir (22) logé de manière mobile dans la base (21) du connecteur de bande (20) et comportant une arête (222) formée dessus et adaptée pour permettre à une bande de buter contre le sommet du poussoir (22) et une protubérance (221) s'étendant dans la base (21) ; et
un doigt rétracteur (23) solidaire de la protubérance (221) du poussoir (22) et monté à coulissement sur un côté du boîtier (10) de telle manière que le mouvement du doigt rétracteur (23) est capable d'entraîner le poussoir (22) pour le déplacer dans la base (21) vers l'entrée (210) de telle façon que la bande présente au sommet du poussoir (22) est aussi déplacée afin de rétracter ladite bande ; et
la carte de circuit (13) est connectée électriquement au moniteur (14) et au connecteur de bande (20).

2. Lecteur biologique selon la revendication 1, dans lequel le doigt rétracteur (23) comporte un élément de liaison (232) qui s'étend depuis le doigt rétracteur (23) et comporte un trou (234) défini dans une extrémité libre de l'élément de liaison (232) et destiné à correspondre avec et à recevoir une extrémité libre de la protubérance (221) du poussoir (22), de sorte que le mouvement du doigt rétracteur (23) est capable d'entraîner le poussoir (22) en mouvement.

3. Lecteur biologique selon la revendication 1, dans lequel le connecteur de bande (20) comporte un espace de glissement (211) défini dans une face inférieure de la base (21) et la protubérance (221) du poussoir (22) est apte à s'étendre hors de l'espace de glissement (211).

4. Lecteur biologique selon la revendication 2, dans lequel le connecteur de bande (20) comporte un espace de glissement (211) défini dans une face inférieure de la base (21) et la protubérance (221) du poussoir (22) est apte à s'étendre hors de l'espace de glissement (211) pour se connecter à l'élément de liaison (232), de sorte que le mouvement du doigt rétracteur (23) entraîne le poussoir (22) en mouvement.

5. Lecteur biologique selon les revendications 1 à 4, dans lequel le connecteur de bande (20) comporte des pattes de contact (212) composées d'une partie s'étendant hors de la base (21) pour effectuer une connexion électrique avec la carte de circuit (13) et une partie logée dans la base (21) destinée à se mettre en contact avec la bande et comportant une partie arquée (213).

6. Lecteur biologique selon la revendication 5, dans lequel le poussoir (22) comporte des évidements (224) définis dans l'arête (222) pour faciliter le mouvement du poussoir (22) relativement aux pattes de contact (212).

7. Lecteur biologique selon la revendication 6, comprenant en outre un ressort de compression (242) adapté pour être mis en butée entre un côté intérieur du boîtier (10) et le doigt rétracteur (23) de telle manière que la force de rappel du ressort de compression (242) due au mouvement du doigt rétracteur (23) est capable de faire revenir le doigt rétracteur (23) dans sa position d'origine.

8. Lecteur biologique selon la revendication 7, dans lequel le doigt rétracteur (23) comporte en outre une tige de guidage s'étendant depuis celui-ci et le ressort de compression (242) est monté autour de la tige de guidage.

9. Lecteur biologique selon la revendication 8, dans lequel une face oblique (214) est formée sur une face périphérique définissant une entrée (210) définie dans la base (21) du connecteur de bande (20) et une face inclinée (223) est formée sur le poussoir (22) de telle manière que l'insertion de la bande dans le lecteur biologique est facilitée.
